# EUROPEAN PATENT APPLICATION

(11) **EP 4 623 951 A1**
(43) Date of publication of application: **01.10.2025**
(21) Application number: 24167158.5
(22) Date of filing: 28.03.2024
(51) Int. Cl.: A61M 5/142, A61M 5/145

(54) **SEMI-DISPOSABLE DRUG DELIVERY DEVICE**

(71) Applicant: TecMed AG, 3400 Burgdorf (CH)
(72) Inventor: Streit, Ursina, 3422 Kirchberg (CH); Baumert, Jan, 3455 Grünen (CH); Buri, Thomas, 3400 Burgdorf (CH); Hanimann, Michael, 3012 Bern (CH); Margot, Roland, 3076 Worb (CH); Steiner, Fabian, 3400 Burgdorf (CH); Heimgartner, Isabelle, 3400 Burgdorf (CH)
(74) Representative: Meier Obertüfer, Jürg

(57) **Abstract**

The present invention is concerned with a drug delivery device, in particular with a wearable patch injector or patch pump for subcutaneous delivery of a fluid medicament from a reservoir. The invention includes an improved design of an interface between a reusable and a disposable portion of the drug delivery device, where a protective lid is attached to the housing of the disposable module to ensure sterility and reliability of drug delivery. The protective lid is ruptured when the reusable module and the disposable module are being connected.

## Description

### FIELD OF THE INVENTION

The present invention relates to a drug delivery device, in particular to a wearable patch injector or patch pump for subcutaneous delivery of a fluid medicament from a reservoir. The invention includes an improved design of an interface between a reusable and a disposable portion of the drug delivery device.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include drug delivery devices that are removed from the injection site after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Departing from classical syringes, increasingly complex devices have been designed to support different therapies, to ensure safety and reliability, and to increase ease of use to a point patients can apply the drugs themselves, reducing time-consuming and costly interventions by trained medical staff to a minimum. Examples of drug delivery devices suitable for self-treatment include injection pens, auto-injectors, portable infusion pumps and wearable patch pumps. Despite of the technical complexity it is an important requirement to keep cost of manufacturing and cost of devices as low as possible.

Common to all devices for subcutaneous drug delivery is a reservoir to store the fluid medicament, typically with a movable plunger sealing the reservoir at a proximal end, and a fluid path to bring the drug out of the device and into the subcutaneous tissue of a patient. Fluid-tightness of the fluid path is an essential requirement to ensure safety and accuracy of the delivery. Longer term infusion patterns and reliable system supervision functions particularly rely on controlled fluid pressure along the fluid path. While this is rather easy to achieve for a classic syringe, it becomes a challenge with increasing complexity of the device. Requirements are further increased by design for self-administration, which means use in a non-sterile environment, use by people without medical training, or even use by people with reduced visual or haptic capacities. The use of pre-filled cartridges, user-friendly fill ports, modular devices with disposable modules as well as wearable devices with auto-inserters are typical solutions to improve ease of use. Reducing the number of mechanical components and design for easy assembly during manufacturing are typical approaches to minimise cost.

US 6,669,668 B1 discloses a drug delivery device with a disposable reservoir and a reusable pump module. The drug is manually filled into the disposable reservoir using a standard syringe. An administration set is used to bring the drug from the pump into the body of the patient.

An important step towards ease of use is to omit the administration set and design a wearable patch pump which is small and has an adhesive patch to attach the pump to the patient during drug delivery. A typical patch pump design has a housing with a reservoir to contain the drug, a cannula to lead the drug into the body of a patient, and a needle assembly to establish a fluid-tight connection between the reservoir and the cannula. For optimum ease of use, the cannula is made of a soft material and an auto-inserting mechanism with a rigid needle or cannula is built into the pump to insert the soft cannula into the body of the patient for drug delivery. For compact and fluid-tight design, the reservoir is generally built into the housing and needs to be filled from outside prior to use. A number of sealing components are needed to ensure a fluid-tight design of the fluid path and of the housing. Special solutions are needed for the fill port, where the drug is brought into the reservoir, and for the exit port, where the cannula passes from the inside of the housing to the outside of the housing for drug delivery. Wearability asks for a compact design of the patch pump as a whole, which further adds to the complexity of design and manufacturing. As the most complex variation of subcutaneous drug delivery devices, semi-disposable patch pumps with internal auto-inserting mechanism and a soft cannula open the door to the most sophisticated therapies at the highest level of ease of use at a potentially low cost. Among other applications, they are a preferred solution for the intermittent delivery of insulin for the treatment of diabetes mellitus.

While a semi-disposable design for a wearable drug delivery device may provide an optimal compromise between ease of use and cost efficiency, the interface between a reusable module and a disposable module is left as a weakness when it comes to safety and reliability of drug delivery. All of the functional requirements in place for the complete pump in an assembled state - like releasable attachment of modules to each other, transfer of mechanical forces or electrical signals between modules, actuations and sensing of any kind of parameters across modules - shall be ensured even if the modules are handled separately before use. As subcutaneous drug delivery always requires sterility of the drug and all components in contact with the drug, ensuring sterility at the interface between the reusable module and the disposable module is a particular challenge.

There is clearly a need for a wearable drug delivery device which provides accurate and reliable drug delivery in an easy to use, robust design which allows keeping therapy cost as low as possible and optimises safety when used in a non-sterile environment.

Sterile primary packaging such as a blister with a removable lid is the standard solution to keep single components or assemblies such as pen needles, infusion sets or fully disposable patch pumps sterile. For semi-disposable drug delivery devices, the disposable module would typically be packed in such a blister, ensuring sterility during transport and storage, but leaving the interface to the reusable module open to contamination during preparation for use. Extra precautional measures such as restricting preparation to qualified health care professionals or teaching patients to wash hands and work on a clean table help reduce the risks, but only to a limited extent.

One way to increase safety is to add an extra protective component, such as a sealing plug over critical openings. This approach is described in WO0069509 A1 (Pescadero Beach Holdings, (200)0). The whole disposable module may be packaged in a sterile blister as a first sealing, while a second sealing inside the blister covers the most critical component of the module, here the reservoir. The second sealing does improve safety, but just like the blister lid the sealing plug needs to be manually removed, adding an extra handling step while still not completely eliminating the risk of contamination.

A more promising approach could be adapted from WO2010/094916 (Oval Medical, 2010). A sealing foil is mounted on the distal opening of the reservoir behind the plunger. A pusher is mounted behind the sealing foil. For drug delivery, the pusher is moved forward, breaks the foil immediately before contacting the plunger, and continues to push the plunger in the reservoir. This approach reduces the risk of contamination prior to drug delivery, even without introducing an extra handling step, because at the time the foil is ruptured the modules are already mechanically connected. However, the risk of contamination is not eliminated, but moved to an other area: parts of the sealing foil may stick to the pusher, get in contact with the plunger, particles of the foil may reach the drug and contaminate the drug itself or affect the sealing function of the plunger. Further, closing the distal opening of the reservoir fixates the plunger inside the reservoir, which prevents filling the reservoir before rupturing the foil and restricts the use of this concept to delivery devices with a pre-filled cartridge.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more ingredients with a medical, therapeutic, diagnostic or other biologic effect. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, dyes, contrast agents, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The term "drug delivery system" or "drug delivery device" refers to a device that is designed to bring any kind of drug as defined above into contact with a patient. This includes injection systems, infusion systems, inhalators and other medical devices used for diagnosis or therapy of both human or animal patients.

### DESCRIPTION OF THE INVENTION

It is an objective of the invention to provide a semi-disposable drug delivery device which is easy to use and ensures sterility of selected disposable parts during all manual handling steps.

This objective is achieved by a mobile or wearable drug delivery device comprising: a reusable module with a reusable housing comprising a first connecting interface; and a disposable module with a disposable housing comprising an actuation opening, a protective lid and a second connecting interface. The first connecting interface and the second connecting interface are configured to removably connect the disposable housing and the reusable housing; the protective lid is adapted to cover at least the actuating opening, providing a sterile barrier for components inside the disposable housing. The disposable module includes a reservoir with a slidable plunger to hold a variable amount of liquid inside the reservoir. The reusable module may further include an actuation assembly configured to be extendable through the actuation opening into the disposable module and to advance the plunger for drug delivery in a state where the two modules are connected. The first connecting interface further includes a rupturing means configured to rupture the protective lid of the disposable module when the reusable module is connected with the disposable module. This arrangement has the effect that the actuation assembly remains outside the actuation opening until the disposable module and the reusable module are properly connected for drug delivery, avoiding contact with the protective lid and hence avoiding risk of contamination by bringing unwanted particles inside the disposable module when advancing for drug delivery. Protecting the inner surface of the reservoir between the step of connecting the pump modules by the user and the step of starting drug delivery by the drug delivery device means that said surface is protected during manual filling when the plunger is pushed in a proximal direction. Avoiding unwanted particles inside the reservoir ensures a maximum achievable sterility of the inner surface of the reservoir and also means that the sealing function of the plunger is ensured over the complete travel inside the reservoir, in both directions, resulting in an improved accuracy and reliability of drug delivery and supervision thereof. Both means and position of a feature to rupture the protective lid can now be chosen freely. The actuation assembly may be extendable through the actuation opening after the reusable module and the disposable module have been connected. In one embodiment, the rupturing means may be radially arranged around the actuation assembly. More generally, the rupturing means may be included in any part of the first or second connecting interface, configured to rupture the protective lid of the disposable module when the disposable module is connected with the reusable module, eliminating the need of an extra handling step. By including the rupturing means in a connecting interface of the housing, either of the disposable module or of the reusable module, or of both modules, no extra components are needed, keeping manufacturing cost as low as possible. Such an improved interface design leads to a variety of novel drug delivery devices which are easy to use and ensure maximum possible sterility of selected disposable parts, in particular the inner surface of the reservoir, during all manual handling steps. The novel drug delivery device is designed to make a user intuitively apply a method to prepare a semi-disposable, mobile or wearable drug delivery device for use, comprising connecting, by the user, a reusable module with a disposable module, characterised by a first connecting interface rupturing a protective lid of the disposable module when the user connects the reusable module with a disposable module.

### BRIEF DESCRIPTION OF THE DRAWINGS

The subject matter of the invention will be explained in more detail in the following text with reference to preferred exemplary embodiments which are illustrated in the attached drawings, in which:

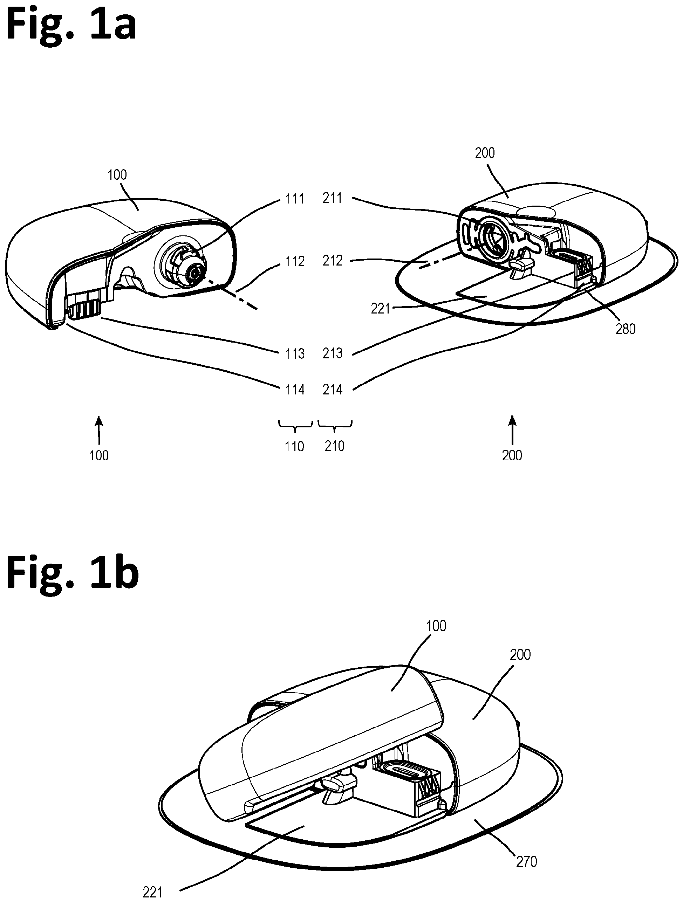

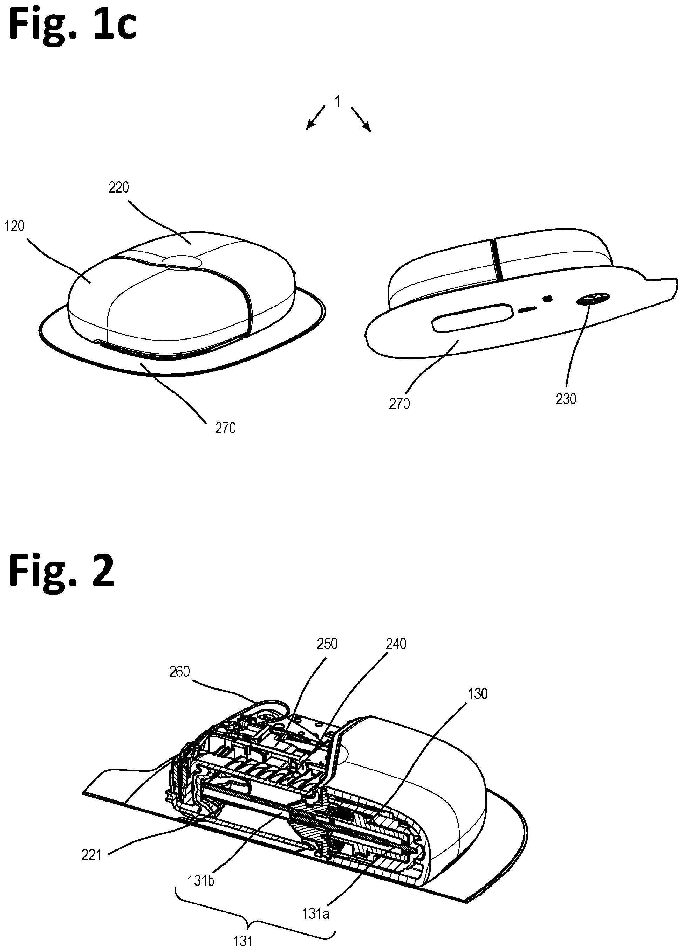

- Fig. 1a, b, c: depicts a patch pump according to the present invention;
Fig. 1a depicts a reusable module (left) and a disposable module (right);
Fig. 1b depicts the patch pump with a reusable module and a disposable module in a connecting position
Fig. 1c depicts the patch pump with a reusable module and a disposable module connected for drug delivery, seen from above (left) and from below (right)
- Fig. 2: depicts a perspective view of the patch pump, cut open and parts of the disposable housing removed to show inner components
- Fig. 3a, b: depict the disposable module with a protective lid attached inside the activation opening, prior to connection with a reusable module;

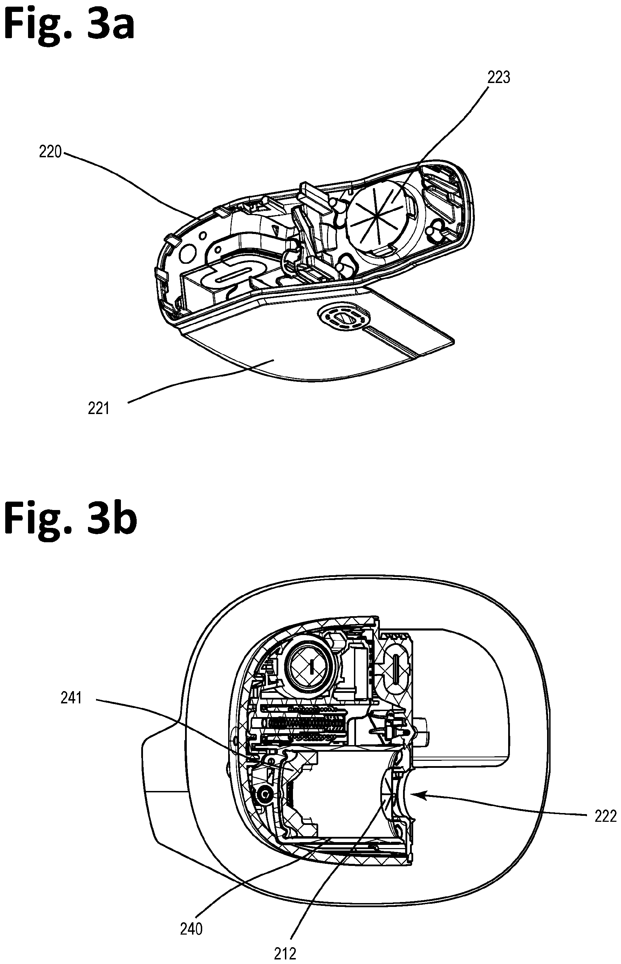

Fig. 3a depicts a perspective view of the disposable module, with most components removed, with a protective lid covers the actuation opening from the inside;
Fig. 3b depicts a cut through the disposable module with a protective lid covering the actuation opening from the inside

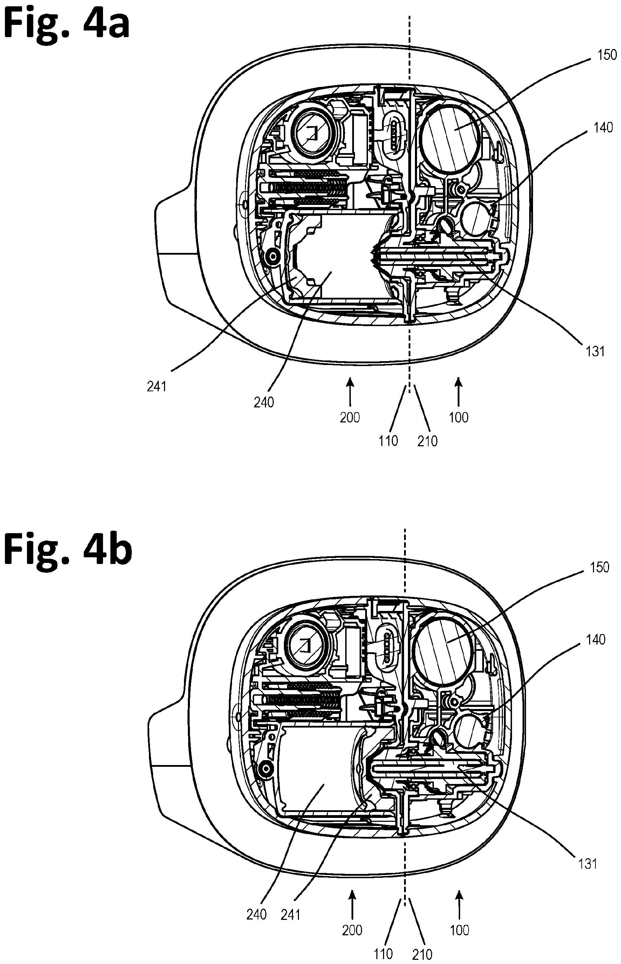

- Fig. 4a, b: depicts a cut through the patch pump after connecting the disposable module with the reusable module, with the protective lid ruptured;
Fig. 4a depicts a cut through the patch pump in a connected state, before filling the reservoir;
Fig. 4b depicts a cut through the patch pump in a connected state, after filling the reservoir

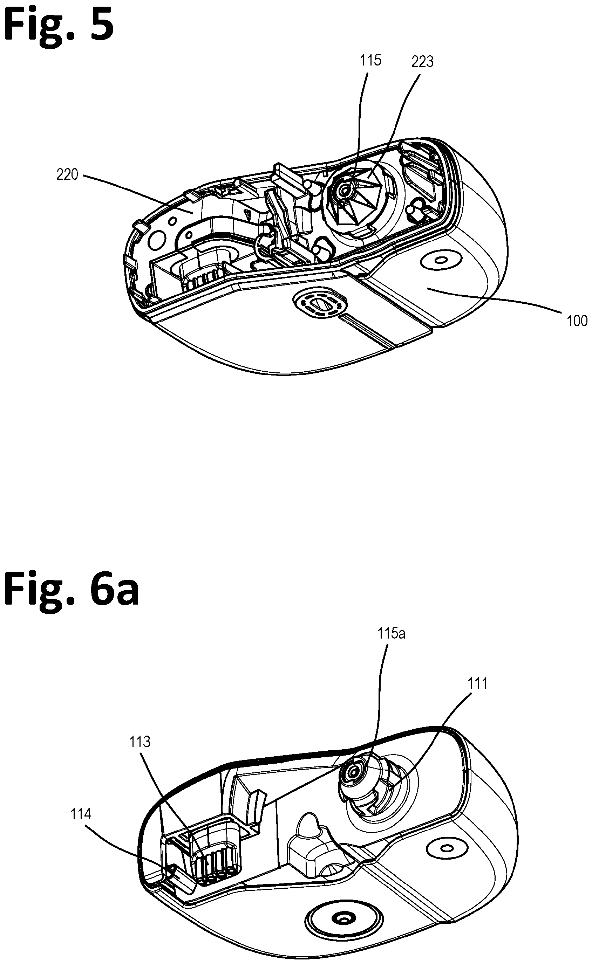

- Fig. 5: depicts the disposable module with the protective lid ruptured, after connection with a reusable module, seen from inside the disposable module

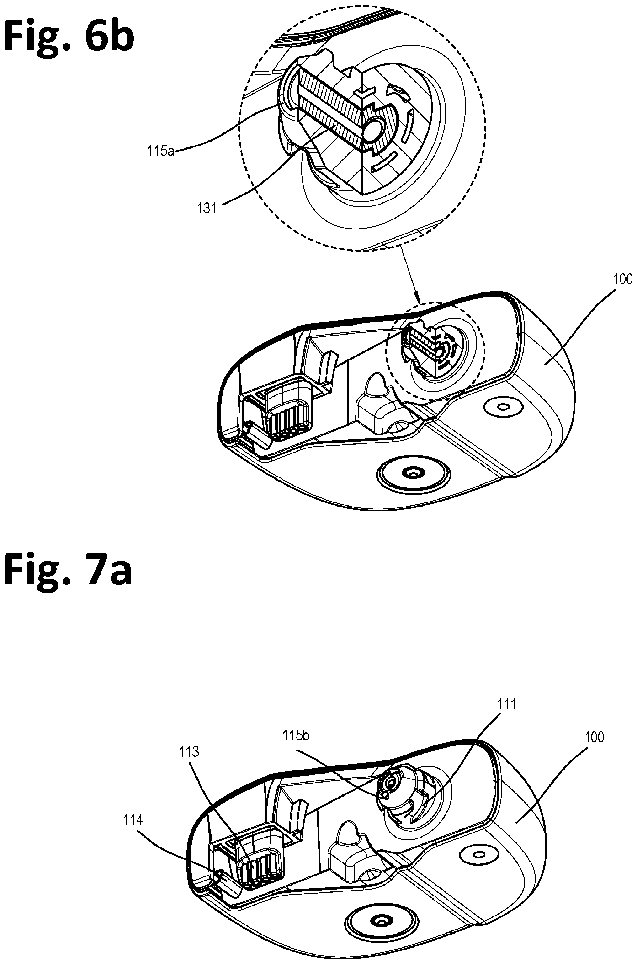

- Fig. 6a, b: depicts the reusable module with a cutting edge as a rupturing means;
Fig. 6a depicts a perspective view of the reusable module with a cutting edge;
Fig. 6b depicts the reusable module of Fig. 6a with a cut and an enlarged view to illustrate the shape of the cutting edge;

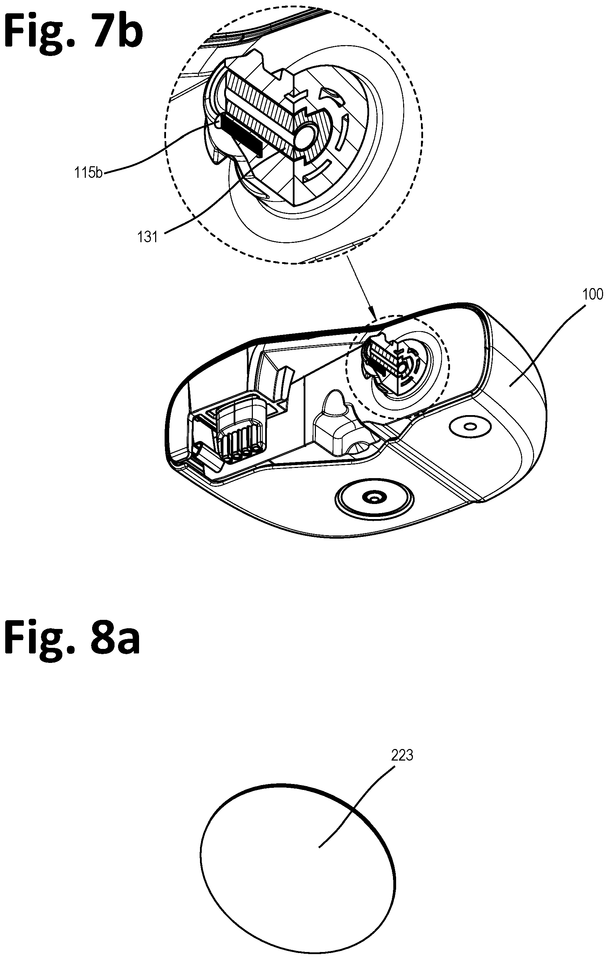

- Fig. 7a, b: depicts the reusable module with a rupturing tip;
Fig. 7a depicts a perspective view of the reusable module with a rupturing tip;
Fig. 7b depicts the reusable module of Fig. 7a with a cut and an enlarged view to illustrate the shape of the rupturing tip

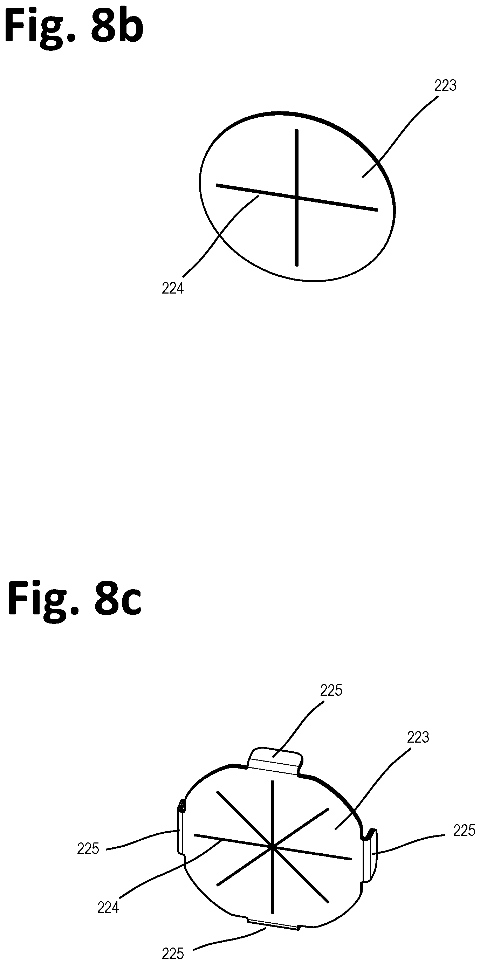

- Fig. 8a, b, c: depicts the protective lid separated from other components;
Fig. 8a depicts a perspective view of a protective lid without weakening structure;
Fig. 8b depicts a perspective view of a protective lid with a cross pattern of weakening grooves;
Fig. 8c depicts a perspective view of a protective lid with four peripheral fixation latches;

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

The present invention is now described using a patch pump as shown in Figure 1 and Figure 2. The drug delivery device is realised as a patch pump 1 attachable to the skin of a patient. The patch pump 1 comprises a reusable module 100 with a reusable housing 120 and a disposable module 200 with a disposable housing 220. The disposable module 200 comprises a reservoir 240 to store the medicament and a needle assembly 260 with a fluid path to bring the drug from the reservoir 240 into the body of the patient. The reservoir 240 may be pre-filled with a liquid drug during manufacturing. Alternatively, the patch pump 1 comprises a filling port 230, typically built into the disposable housing 220. Through such a filling port 230, an external syringe can be used to to reach through the disposable housing 220 to the distal end of the reservoir 240 and press a liquid drug into the reservoir 240. At the bottom of the disposable module 200 an adhesive patch assembly 270 is included to attach the patch pump 1 to the body of the patient. The reusable module 100 is releasably and sealingly connected to the disposable module 200 by a set of two matching connecting interfaces, a first connecting interface 110 on the reusable module 100 and a second connecting interface 210 on the disposable module 200. Various features may be included in the connecting interfaces to realise all sorts of functions across the connection and allow the patch pump to work as a complete drug delivery device. Features may for example include mechanical, electrical, optical, hydraulic, electromagnetic or fluidic connections; functions may for example include mechanical attachment, locking/unlocking, guidance, communication, protection, indication, sensing or actuating. A few typical features are shown in Figure 1a, where a first connecting interface 110 on a reusable module 100 is depicted in a separated state from a matching second connecting interface 210 on a disposable module 200. In this exemplary embodiment, the mechanical fixation of the modules is realised using a bayonet connection 111, 211. Electric contacts 113, 213 provide the means for transmitting electric power and signals to control the patch pump. Figure 1b illustrates how the reusable module 100 is aligned with the disposable module along the rotation axis 112, 212 of the bayonet connection 111, 211, ready to be rotated down onto the base plate 221 of the disposable module 200. A locking mechanism such as a mechanical latch 114, 214 is typically included in the connecting interface to hold the modules in a connected state for drug delivery. The connected state is shown in Figure 1c. The reusable module 100 comprises a drive mechanism 130 for driving an actuation assembly 131, an encoder to supervise the movement of the drive mechanism, a rechargeable battery 150 and a control unit 140 configured to control the set-up, drug delivery and supervision of the pump. The actuation assembly 131 may comprise any number of operatively connected components to advance the plunger 241, in particular a threaded rod 131a and a plunger rod 131b. The disposable module 200 comprises a reservoir 240 with a plunger 241, and an inserter assembly 250 operatively connected to a needle assembly 260. The needle assembly 260 may include a rigid cannula, or a soft cannula and a rigid cannula operatively connected to the soft cannula for insertion as described above. The reservoir may be filled with a liquid drug before or after connecting the two modules. With the patch pump 1 connected and the reservoir 240 filled with liquid drug, the control unit 140 may trigger the insertion of at least a portion of the needle assembly 260 into the body of the patient, activate the drive mechanism 130 in the reusable module 100 to extend the plunger rod 122 through an actuation opening 222 into the disposable module 200, operatively contact the plunger 241, continue driving and effectuate drug delivery from the reservoir 240 via the needle assembly 260 into the body of the patient. The effect of the semi-disposable concept of Figure 1 reduces the number of handling components to a minimum and thus provides a semi-disposable drug delivery device which is easy to use and the best basis for further improvement. It is an important further aspect of this invention that the patch pump of Figure 1 is improved by adding a protective lid 223 covering at least part of the second connecting interface 210. In Figure 3, such a protective lid 223 is shown fixedly attached to the inner face of a component comprised in the disposable housing 220. In this example, the protective lid 223 covers the actuation opening 222. The protective lid 223 does not close the proximal opening of the reservoir 240 and hence provides full protection while the plunger 241 still may move freely inside the reservoir 240. The reservoir 240 may be filled with liquid drug while the protective lid remains intact and the reservoir 240 fully protected prior to connecting the reusable module 100 with a disposable module 200. Figure 4 depicts a cut through the complete patch pump after connecting the two modules to indicate the location of the protective lid 223. In the embodiment shown in Figure 4 the disposable module 200 is manufactured with an empty reservoir 240, leaving the process of filling to a user, be it the patient receiving the drug or any other person preparing the drug delivery device. The plunger 241 is in a position opposite to the actuation opening 222. By covering at least the actuation opening 222, the protective lid 223 prevents the inner surface of the reservoir 240 and the plunger 241 from being contaminated before filling the reservoir 240 with the liquid drug, and from affecting the sealing function of the plunger 241. Consequently, the protective lid 223 prevents the liquid drug from being contaminated during all handling steps up to start of drug delivery.

While covering an opening in the disposable housing with a protective lid does indeed improve safety and reliability, such a lid needs to be opened for use. Therefore, along with the protective lid 223, a puncturing or rupturing means 115 needs to be included to enable proper function of the drug delivery device. It is an important aspect of the present invention that puncturing or rupturing of any number of protective lids takes place during the one handling step of connecting the reusable module 100 with the disposable module. After puncturing or rupturing the protective lid, all other mechanical, electrical, optical, hydraulic, electromagnetic or fluidic features included in the connecting interface may hence be used without compromise to fulfil their specified function in guidance, communication, indication, sensing or actuating, and still benefit from the effect of the protective lid 223. This is especially advantageous for the function of advancing a part of the actuation assembly 131, for example the plunger rod 131b through the actuation opening 222 into the disposable module 200.

The principle described for the embodiments of Figures 1 to 4 may be applied to any drug delivery device, for example a tubeless patch infusion pump attachable to the skin of a patient by means of an adhesive layer, for subcutaneous delivery of insulin through a cannula integrated into the pump over a period of more than 48 hours. Alternatively, the delivery device is not a tubeless patch infusion pump attachable to the skin of a patient by means of an adhesive layer, for subcutaneous delivery of insulin through a cannula integrated into the pump over a period of more than 48 hours. For instance, the alternative delivery device may be patch injector attachable to the skin of a patient by means of an adhesive layer, for subcutaneous delivery of a drug other than insulin over a period of less than 48 hours. The alternative delivery device may be a wearable insulin pump or a handheld injection pen devoid of an adhesive layer for attaching to the skin of a patient.

Still using Figures 3 and 4, more details are added about the position and design of the protective lid 223. As such a lid is added to protect the inside of the disposable module from ingress of contamination or particles, all openings of the disposable module may be chosen to be covered by a protective lid. There may be a plurality of protective lids, connected or not, movably or fixedly attached to any part of the disposable module. In Figure 3a, one example of an embodiment is shown where the protective lid 223 is attached to the inner face of the disposable housing 220, covering the actuation opening 222. Figure 3a gives a perspective view as seen from the inside of the disposable module, with most parts removed to see the shape and position of the protective lid 223. Having the protective lid 223 on the inner face of the disposable housing 220 rather than the outside of the disposable housing 220 has the effect that the protective lid 223 does not affect the bayonet connection 111, 211 and vice versa. It may be desirable for some applications to combine a feature of the mechanical connection mechanism - in this case: the bayonet connection 111 - with the rupturing means 115, but the bayonet connection 111, 211 may not always be located at a position most critical for contamination, therefore for illustration purposes an example has been chosen where the rupturing means 115 is separated from the reusable side of the bayonet connection 111.

Figure 3b shows the example from Figure 3a in a cut through the disposable module 200, in a state before being connected with the reusable module 100. While the protective lid 223 is intact and clearly closes the actuation opening 222, the protective lid 223 has no connection with the reservoir 240. This means that, while providing protection from contamination and ingress of external components, the protective lid 223 does not close the proximal opening of the reservoir housing 230, avoids excess air pressure behind the plunger and hence leaves the plunger 241 free to move inside the reservoir housing 230 when the reservoir is being filled from a distal end using an external syringe. It is a further aspect of the invention that a maximum of sterility can be ensured regardless of whether the reservoir 240 of the patch pump 1 is filled before or after connection of the reusable module 100 and the disposable module 200. Figure 4 illustrates how the plunger 241 can be moved inside the reservoir 240 from a state with an empty reservoir (Figure 4a) to a state with a filled reservoir (Figure 4b) without moving the actuation assembly 131. The handling step of connecting the reusable module 100 with the disposable module 200 includes establishing a mechanical connection between the first connecting interface 110 and the second connecting interface 210. In the example of Figure 1, the mechanical connection is realised by a bayonet connection 111, 211. Any part of the reusable module 100 may be configured to rupture the protective lid 223 when connecting, closing or locking the bayonet connection 111, 211. From a user's point of view, the handling step of connecting a disposable module 200 and a reusable module 100 hence includes rupturing the protective lid 223 of the disposable module 200. There may be multiple parts of the reusable module involved in rupturing any number of protective lids, regardless of where on the second connecting interface a part of such a protective lid may be positioned. In the example shown in Figure 4, the rupturing means 115 is radially arranged around the actuation assembly 131, covering the distal end of the plunger rod 122 so that the protective lid 223 is not touched by the actuation assembly 131 in any situation: not when the user is connecting the modules, not when the protective lid 223 is ruptured, and not when the actuation assembly is advanced through the actuation opening for drug delivery. This fact further contributes to ensuring sterility and avoiding ingress of external components into the disposable module and to the inside of the reservoir. With external components efficiently excluded, internal components, in particular components breaking off the protective lid when the protective lid 223 is ruptured, come into the focus. To avoid contamination or loss of sealing of the plunger, the protective lid 223 is made of a homogenous and flexible or elastic material free of fibers. The degree of flexibility or elasticity needs to be adapted to the shape of both the protective lid 223 and the rupturing means 115. All sorts of combinations of the two components are possible, such as a sharp rupturing tip piercing a highly elastic rubber membrane assembled under tension and retracting to a relaxed state, or a blunt rupturing tip moving a moderately flexible membrane out of the way. A foil or heat sealing film made of a plastic polymer such as polyethylene (PE) is a good example of such a moderately flexible material. Inhomogeneous materials, in particular materials containing fibers, are prone to lose particles when being ruptured and hence are less suited for use as such a protective lid. A sheet made of an elastic material such as silicon or rubber may be used as a protective lid in an alternative embodiment, where the rupturing means 115 is creating an opening in the protective lid 223. Depending on the requirements of the connecting interface, a small opening may suffice to establish an electrical, optical, hydraulic or fluidic connection and allow functions such as mechanical attachment, locking/unlocking, guidance, communication, indication, sensing or actuating. It is a further aspect of the invention that rupturing a protective lid may be supported by introducing a weakening structure 224 to at least one surface of at least part of a protective lid 223. The weakening structure may include a groove realised by deformation, cutting or scratching a surface of the protective lid 223. The weakening structure 224 provides a mechanical weakening of the protective lid 223, typically by thinning out the material along a predefined line, without cutting through the lid. Many forms of weakening are well known and can be applied in the context of the improved drug delivery device described in this document. It is equally well known that a protective lid 223 with a weakening structure 224 may also be manufactured by using a thin basic sheet and partially adding a reinforcement structure, for example a coating or an extra layer of any material, resulting in a mechanical equivalent of a protective lid 223 with a weakening structure 224.

The weakening structure 224 has the effect of defining where and how the protective lid 223 will rupture, and of reducing the force necessary to effectuate the rupturing. The weakening structure 224 helps avoiding particles to become loose and improves both protection from contamination, sterility and ease of use. Examples of weakening structures 224 are best visible in Fig. 3a, Fig.8b and Fig 8c. Such a weakening structure may be applied to just one surface of a protective lid 223, or on both surfaces, the inner surface towards the inside of the disposable module 200 and the outer surface towards the outside of the disposable module 200. Weakening structures may have any geometrical shape, such as a simple crossing of two lines (Fig. 8b), crossing of more than two lines (Fig. 8c), circular shapes, or any shape following the contour of an opening in the disposable module 200.

In order to ensure that the protective lid 223 is ruptured, at least part of the protective lid 223 needs to be fixedly attached to or integrated into the housing of the disposable module 200. This makes sure that the rupturing means 115 actually ruptures the protective lid 223 rather than just separating the protective lid from the disposable housing 220. Any kind of fixation technique may be used to realise the attachment of the protective lid 223 to the disposable housing 220, for example glueing, soldering, injection molding, heat stacking, welding and crimping. Some of the techniques, such as glueing, may need a bigger surface area to ensure sufficient fixation than available around the opening in the disposable housing 220. In these cases, the protective lid 223 may include one or more lateral extensions 225 where the fixation can be realised or reinforced. In another embodiment, the weakening structure 224 of a protective lid 223 may be realised by omitting such an extension or weakening the fixation of a protective lid 223 on the disposable housing 220, resulting in a controlled partial or complete removal of a protective lid 223 from an opening in the disposable housing, which also will help avoiding contamination by particles set free by rupturing the protective lid.

Figure 5 shows an embodiment with a protective lid 223 made of flexible PE. Using a view from inside the disposable module in a connected state, with selected parts removed as in Figure 3a, the protective lid 223 is shown in a ruptured state with the rupturing means 115 visible in the actuation opening. Figure 6 illustrates in more detail how the rupturing means 115 is realised as part of the first connecting interface for this embodiment. Figure 6a gives a perspective view of the reusable module with a rupturing means, Figure 6b a cut through such a reusable module. If rupturing involves cutting the protective lid - as the case in the example of the PE foil - a cutting edge 115a would be arranged at the distal end of the rupturing means, as shown in Figure 6. As clearly shown in the detail of Figure 6b, the cutting edge 115a of the rupturing means 115 is protruding from the end of the actuation assembly 131 and therefore rupturing the protective lid 223 without need of the actuation assembly coming into contact with the protective lid 223. While the cutting edge 115a is shown in Figure 6a as a circular ring, such an edge may be configured to have any geometric form. In an other embodiment, the rupturing means may include a pointed or blunt piercing tip 115b as shown in Figure 7.

The novel drug delivery device described above is designed to make a user intuitively apply a method to connect a disposable module and a reusable module to form a drug delivery device, whereby a first connecting interface is configured to rupture a protective lid of the disposable module when the user connects the reusable module with a disposable module. The sequence of these steps ensures an improvement in sterility and reliability of drug delivery, as the protective lid 223 remains intact for as long as possible, including preparation of the reusable module 100 and the disposable module 200 for use, and including filling of the reservoir 240. Rupturing or at least partially removing the protective lid 223 from the disposable housing 220 allows and enables all sorts of functions possible across the connecting interface of the reusable module 100 and the disposable module 200, be it mechanical, electrical, optical, hydraulic, electromagnetic or fluidic. In one embodiment, rupturing of a protective lid 223 enables advancing at least part of the actuation assembly 131 from the reusable module 100 through the actuation opening 222 into the disposable module 200, for example the distal end of the plunger rod 131b.

In some embodiments, the combination of protective lid 223 and rupturing means 115 is configured not to entirely rupture the protective lid 223 when connecting the reusable module 100 with the disposable module 200, but to weaken or bias the protective lid 223. When advancing the plunger rod 122 or another part of the actuation assembly 131, the protective lid 223 will snap or be pushed away, resulting in a similar effect of reducing risk of contamination and improving reliability of a drug delivery device as if the protective lid 223 would have been ruptured prior to advancing the plunger rod 122.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Patch pump or delivery device
- 100: Reusable module
- 110: First connecting interface
- 111: Bayonet connection
- 112: Rotation axis
- 113: Electric contacts
- 114: Mechanical latch or locking mechanism
- 115: Rupturing means
- 115a: Cutting edge
- 115b: Piercing tip
- 120: Reusable housing
- 130: Drive mechanism
- 131: Actuation assembly
- 131a: Threaded rod
- 131b: Plunger rod
- 140: Control unit
- 150: Rechargeable battery
- 200: Disposable module
- 210: Second connecting interface
- 211: Bayonet connection
- 212: Rotation axis
- 213: Electric contacts
- 214: Mechanical latch or locking mechanism
- 220: Disposable housing
- 221: Base plate
- 222: Actuation opening
- 223: Protective lid
- 224: Weakening structure
- 225: Extension
- 230: Filling port
- 240: Reservoir, reservoir housing
- 241: Plunger
- 250: Inserter assembly
- 260: Needle assembly
- 270: Adhesive patch assembly

## Claims

1. A mobile or wearable drug delivery device (1) comprising
a reusable module (100) with a reusable housing (120) comprising a first connecting interface (110),
a disposable module (200) with a disposable housing (220) comprising an actuation opening (222), a protective lid (223) and a second connecting interface (210);
whereby the first connecting interface (110) and the second connecting interface (210) are configured to removably connect the disposable housing (220) and the reusable housing (120);
whereby the protective lid (223) is adapted to cover at least the actuating opening (222), providing a sterile barrier for components inside the disposable housing (220);
whereby the disposable module (200) includes a reservoir (240) with a slidable plunger (241) to hold a variable amount of liquid inside the reservoir (240);
whereby the reusable module (100) includes an actuation assembly (131) configured to be extendable through the actuation opening (222) into the disposable module (200) and advance the plunger (241) for drug delivery in a state where the two modules are connected;
***characterised by*** the first connecting interface (110) further including a rupturing means (115) configured to rupture the protective lid (223) of the disposable module (200) when the reusable module (100) is connected with the disposable module (200).

2. The drug delivery device according to claim 1, whereby the actuation assembly (131) is extended through the actuation opening (222) after the reusable module (100) and the disposable module (200) have been connected.

3. The drug delivery device according to claim 2, whereby the rupturing means (115) is radially arranged around the actuation assembly (131).

4. The mobile or wearable drug delivery device according to claim 1, whereby the protective lid (223) is made of a homogenous and flexible or elastic material free of fibers.

5. The mobile or wearable drug delivery device according to claim 4, whereby the protective lid (223) is a foil made of a plastic polymer such as polyethylene (PE).

6. The mobile or wearable drug delivery device according to claim 4, whereby the protective lid (223) is a sheet made of an elastic material such as silicon or rubber.

7. The mobile or wearable drug delivery device according to claim 4, whereby at least one surface of the protective lid (223) includes a weakening structure (224) or a layer shaped to provide the mechanical equivalent of such a weakening structure (224).

8. The mobile or wearable drug delivery device according to claim 7, whereby the weakening structure (224) includes a groove realised by deformation, cutting or scratching a surface of the protective lid (223).

9. The mobile or wearable drug delivery device according to claim 4, whereby the protective lid (223) includes at least one extension (225) to fixate the protective lid (223) to a part of the disposable housing (220).

10. The mobile or wearable drug delivery device according to claim 2, whereby the rupturing means (115) includes a cutting edge (115a) formed by a part of the reusable housing (120).

11. The mobile or wearable drug delivery device according to claim 2, whereby the rupturing means (115) includes a piercing tip (115b) formed by a part of the reusable housing (120).

12. A method to prepare a semi-disposable drug delivery device according to claim 1 for use, comprising connecting, by the user, the reusable module (100) with the disposable module (200), ***characterised by*** the step of the first connecting interface (110) rupturing the protective lid (223) of the disposable module (200) when the user connects the reusable module (100) with the disposable module (200).

13. The method of claim 12, whereby the rupturing of a protective lid (223) enables a mechanical, electrical, optical, hydraulic, electromagnetic or fluidic function of the connecting interface (110, 210).

14. The method of claim 13, whereby rupturing of a protective lid (223) enables advancing at least part of the actuation assembly (131) through the actuation opening (222) into the disposable module (200).

15. The method of claim 12, whereby the plunger rod (131b) is advanced through the actuation opening (222) into the disposable module (200).
